(19) Europäisches Patentamt / European Patent Office / Office européen des brevets

(11) **EP 4 643 760 A1**

(12) **EUROPEAN PATENT APPLICATION**
published in accordance with Art. 153(4) EPC

(43) Date of publication:
**05.11.2025 Bulletin 2025/45**

(21) Application number: **23911244.4**

(22) Date of filing: **10.08.2023**

(51) International Patent Classification (IPC):
*A61B 3/06* (2006.01)        *G02C 7/02* (2006.01)
*G02C 7/06* (2006.01)        *G02C 13/00* (2006.01)

(52) Cooperative Patent Classification (CPC):
A61B 3/06; G02C 7/02; G02C 7/06; G02C 13/00

(86) International application number:
**PCT/JP2023/029216**

(87) International publication number:
**WO 2024/142460 (04.07.2024 Gazette 2024/27)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **26.12.2022 JP 2022207725**

(71) Applicant: **Nikon-Essilor Co., Ltd.
Tokyo 130-0026 (JP)**

(72) Inventor: **CHO, Sungjin
Tokyo 130-0026 (JP)**

(74) Representative: **Viering, Jentschura & Partner
mbB
Patent- und Rechtsanwälte
Grillparzerstraße 14
81675 München (DE)**

(54) **EYEGLASS LENS PRODUCTION METHOD, EYEGLASS LENS ORDERING DEVICE, EYEGLASS LENS ORDER RECEIVING DEVICE, AND EYEGLASS LENS ORDERING AND ORDER RECEIVING SYSTEM**

(57) Contrast sensitivity of a wearer of an eyeglass lens (10) is measured using a first eye chart and a second eye chart with different spatial frequencies, and the eyeglass lens (10) is designed using a design parameter based on the contrast sensitivity of the wearer. For example, the eyeglass lens (10) is designed such that a distribution of an addition power in a progressive portion of the eyeglass lens (10) becomes high, that is, a refractive power of the progressive portion becomes high as a difference (rate of change) in contrast sensitivity with respect to the different spatial frequencies becomes large.

*FIG.2*

EP 4 643 760 A1

**Description**

TECHNICAL FIELD

The present invention relates to an eyeglass lens production method, an eyeglass lens ordering device, an eyeglass lens order receiving device, and an eyeglass lens ordering and order receiving system.

TECHNICAL BACKGROUND

A method for using visual acuity and contrast sensitivity is known as a method for evaluating and investing a person's eyesight (for example, see Patent Literature 1). It is known that the contrast sensitivity is different depending on individual differences, but an eyeglass lens has not been designed taking into account individual differences in contrast sensitivity.

PRIOR ARTS LIST

PATENT DOCUMENT

Patent Literature 1: Republished International Patent Publication No. WO 2021/157001

SUMMARY OF THE INVENTION

An eyeglass lens production method according to the present invention comprises: performing measurement of contrast sensitivity of a wearer of an eyeglass lens using at least two eye charts with different spatial frequencies; performing a design of the eyeglass lens based on a measurement result of the contrast sensitivity; and producing the eyeglass lens based on the design.

An eyeglass lens ordering device according to the present invention comprises: an input part that inputs information on a measurement result of contrast sensitivity of a wearer of an eyeglass lens which performs measurement using at least two eye charts with different spatial frequencies, or a design parameter calculated based on the information; and a transmitting part that transmits the information input through the input part or the design parameter to an eyeglass lens order receiving device.

An eyeglass lens order receiving device according to the present invention comprises: a receiving part that receives information on a measurement result of contrast sensitivity of a wearer of an eyeglass lens which performs measurement using at least two eye charts with different spatial frequencies, or a design parameter calculated based on the information; and a design part that performs a design of an eyeglass lens based on the information or the design parameter.

An eyeglass lens ordering and order receiving system according to the present invention comprises: the eyeglass lens ordering device described above; and
the eyeglass lens order receiving device described above.

BRIEF DESCRIPTION OF THE DRAWINGS

FIG. 1 is a schematic diagram showing a pair of eyeglass lenses according to the present embodiment;
FIG. 2 is a diagram showing a relationship between a vertical position on an eyeglass lens and addition power;
FIG. 3 is a graph showing a relationship between a distance to an eye chart and an apparent spatial frequency of the eye chart;
FIG. 4 is a block diagram showing an eyeglass lens ordering and order receiving system;
FIG. 5 is a flowchart showing a flow of a method for producing a pair of eyeglass lenses;
FIG. 6 is a diagram showing an example of an order screen;
FIG. 7 is a flowchart showing a flow of a method for measuring contrast sensitivity;
FIG. 8 is a conceptual diagram showing an example of obtaining a minimum eye chart visible to a wearer;
FIG. 9 is a conceptual diagram showing an example of performing a first measurement of contrast sensitivity;
FIG. 10 is a conceptual diagram showing an example of performing a second measurement of contrast sensitivity;
FIG. 11 is a block diagram showing a measuring device;
FIG. 12 is a diagram showing an example of an image of a reference eye chart;
FIG. 13 is a diagram showing an example of an image of a first eye chart;

FIG. 14 is a diagram showing an example of an image of a second eye chart;
FIG. 15 is a diagram showing a modified example of an image of a reference eye chart;
FIG. 16 is a diagram showing modified examples of images of first and second eye charts;
FIG. 17 is a graph showing an example of an addition power in an eyeglass lens;
FIG. 18 is a graph showing an example in which the addition power in the eyeglass lens is changed; and
FIG. 19 is a flowchart showing a flow of a method for designing an eyeglass lens.

DESCRIPTION OF THE EMBODIMENTS

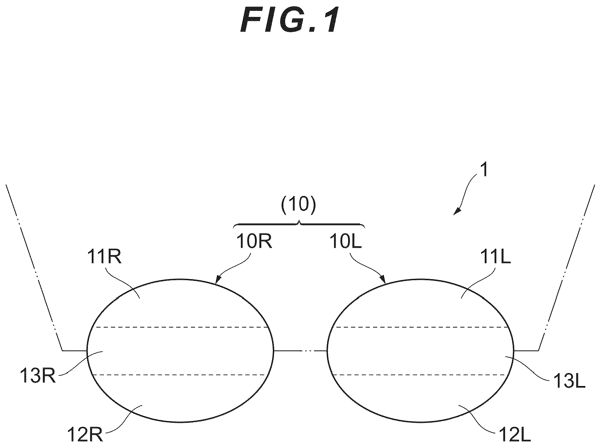

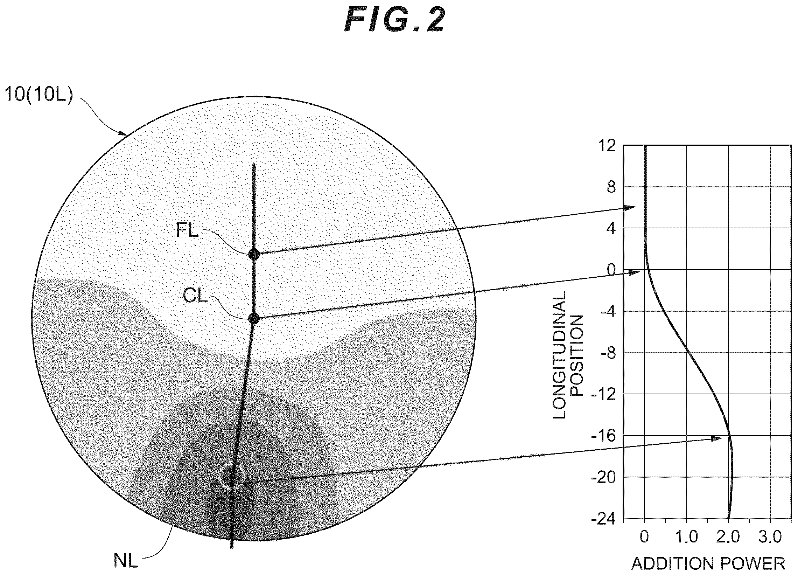

A preferred embodiment of the present invention will be described below. FIG. 1 schematically shows a pair of eyeglass lenses 1 of the present embodiment. As shown in FIG. 1, the pair of eyeglass lenses 1 comprises a right-eye eyeglass lens 10R used for a right eye ER and a left-eye eyeglass lens 10L used for a left eye EL. In the present embodiment, the right-eye eyeglass lens 10R and the left-eye eyeglass lens 10L may be collectively referred to simply as the eyeglass lens 10. The eyeglass lens 10 is also referred to as a progressive refractive power lens. A positional relationship of an "upper" and a "lower" in the eyeglass lens 10 indicates a positional relationship when the eyeglass lens 10 is processed for eyeglass lenses and the eyeglass lenses are worn. The upper and lower positional relationship of the eyeglass lens 10 is assumed to coincide with an upper and lower positional relationship on paper surfaces of FIGS. 1 and 2.

As shown in FIG. 1, the right-eye eyeglass lens 10R comprises a right-eye distance portion 11R, a right-eye near portion 12R formed in a different position from the right-eye distance portion 11R, and a right-eye progressive portion 13R formed between the right-eye distance portion 11R and the right-eye near portion 12R. For example, the right-eye distance portion 11R is formed in an upper part of the right-eye eyeglass lens 10R, the right-eye near portion 12R is formed in a lower part of the right-eye eyeglass lens 10R, and the right-eye progressive portion 13R is formed in an intermediate part of the right-eye eyeglass lens 10R. The right-eye distance portion 11R has a refractive power suitable for distance viewing. The right-eye near portion 12R has refractive power suitable for near viewing. The right-eye progressive portion 13R is configured to have a refractive power changing continuously from the refractive power suitable for distance viewing to the refractive power suitable for near viewing as going from a side closer to the right-eye distance portion 11R toward a side closer to the right-eye near portion 12R. In the present embodiment, a "dioptric power" (unit: diopter [D]) may be used as a numerical value representing a refractive power. A power specified by a prescribed value is referred to as a "prescribed power". A power change relative to a distance power (a refractive power suitable for distance viewing) is referred to as an "addition power".

As shown in FIG. 1, the left-eye eyeglass lens 10L comprises a left-eye distance portion 11L, a left-eye near portion 12L formed in a different position from the left-eye distance portion 11L, and a left-eye progressive portion 13L formed between the left-eye distance portion 11L and the left-eye near portion 12L. For example, the left-eye distance portion 11L is formed in an upper part of the left-eye eyeglass lens 10L, the left-eye near portion 12L is formed in a lower part of the left-eye eyeglass lens 10L, and the left-eye progressive portion 13L is formed in an intermediate part of the left-eye eyeglass lens 10L. The left-eye distance portion 11L has a refractive power suitable for distance viewing. The left-eye near portion 12L has a refractive power suitable for near viewing. The left-eye progressive portion 13L is configured to have a refractive power continuously changes from the refractive power suitable for distance viewing to the refractive power suitable for near viewing as going from a side closer to the left-eye distance portion 11L toward a side closer to the left-eye near portion 12L.

A plurality of reference points is set on the eyeglass lens 10. For example, as shown in FIG. 2, a plurality of reference points, for example, an optical center CL, a distance reference point FL, and a near reference point NL is set on the left-eye eyeglass lens 10L. The optical center CL is a reference point that is a center in the design. The distance reference point FL is a measurement reference point during measurement of a distance power (a refractive power suitable for distance viewing) of the left-eye distance portion 11L. The near reference point NL is a measurement reference point during measurement of a near power (a refractive power suitable for near viewing) of the left-eye near portion 12L. Although not shown, a plurality of reference points, for example, an optical center, a distance reference point, and a near reference point is also set on the right-eye eyeglass lens 10R, similarly to the left-eye eyeglass lens 10L.

As described above, in the eyeglass lens 10 called a progressive-power lens, the distance portion having the refractive power suitable for distance viewing is formed in the upper part of the eyeglass lens 10, and the near portion having the refractive power suitable for near viewing is formed in the lower part of the eyeglass lens 10. The refractive power changes smoothly from the distance portions to the near portions by adding a predetermined addition power to the distance power. The distance power and the addition power are determined by an eye examination. How to distribute the addition power in the intermediate portion (progressive portion) between the distance portion and the near portion is an important element in the design of the progressive-power lens.

FIG. 2 shows an example of a change in addition power with respect to a vertical change in line of sight in the eyeglass lens 10 (for example, the left-eye eyeglass lens 10L). A vertical axis of the graph in FIG. 2 indicates a vertical position [mm] along a straight line in the eyeglass lens 10 showed in FIG. 2. Hereinafter, the vertical position will be referred to as a longitudinal position in the eyeglass lens 10. A horizontal axis of the graph in FIG. 2 indicates addition powers [D] at respective longitudinal positions in the eyeglass lens 10. As shown in FIG. 2, the addition power at the distance reference point of the eyeglass lens 10 (for example, the distance reference point FL of the left-eye eyeglass lens 10L) is 0 (zero), and the addition power gradually increases toward the near reference point of the eyeglass lens 10 (for example, the near reference point NL of the left-eye eyeglass lens 10L). When an accommodative force of eyes is ignored and only the refractive power of the eyeglass lens 10 is considered, a clearly visible distance through the eyeglass lens 10 is determined according to the addition power. When the addition power is small, a long-distance object (an object to be viewed) can be clearly viewed through the eyeglass lens 10, and when the addition power is large, a short-distance object (an object to be viewed) can be clearly viewed through the eyeglass lens 10.

In addition, when an eye chart made up of letters or the like is viewed, even in the eye chart with the same size, the eye chart appears relatively large (or coarse) when the distance to the eye chart is close, and the eye chart appears relatively small (or fine) when the distance to the eye chart is far. In the present embodiment, the spatial frequency is defined as the number of cycles of a sinusoidal grating included in an angle range of 1° centered on a position of eyes (for example, an eye point or a center point of rotation). When letters (for example, Snellen letters, Sloan letters, the letter "E" on an E chart, and the letter "C" on a Landolt ring) are used as the eye chart, a width of lines constituting the letter is regarded as half a wavelength of a sine wave, and the spatial frequency of the eye chart is calculated. In the Snellen letters, the Sloan letters, the letter "E" on the E chart, and the letter "C" on the Landolt ring, the width of the lines and a distance between the lines that constitute the letters have fixed values.

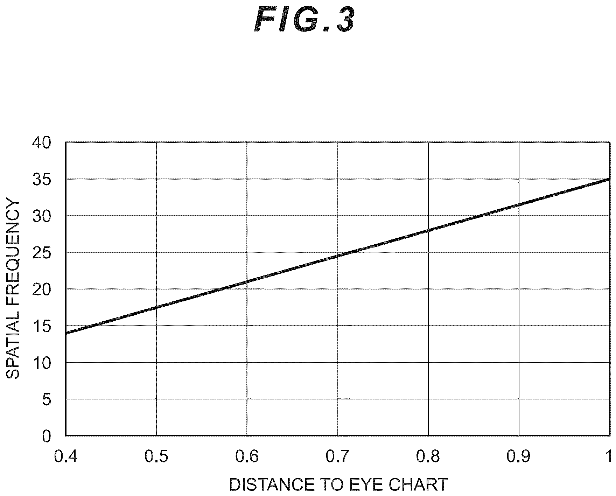

FIG. 3 shows an example of a change in a spatial frequency of the eye chart with respect to a change in a distance to the eye chart. A horizontal axis of the graph in FIG. 3 indicates a distance [m] from an eyeball to the eye chart. A vertical axis of the graph in FIG. 3 indicates a spatial frequency [cycle/deg] of the eye chart. As shown in FIG. 3, even in the eye chart with the same size, the spatial frequency of the eye chart is low when the distance to the eye chart is close, and the spatial frequency of the eye chart is high when the distance to the eye chart is far. Therefore, when the addition power is large at the same position on the eyeglass lens, the clearly visible distance through the eyeglass lens is short, and thus a spatial frequency of the object (object to be viewed) clearly viewed through the eyeglass lens will be low.

In addition, contrast sensitivity to the eye chart varies depending on the spatial frequency of the eye chart. Assuming that the maximum luminance of the eye chart is Lmax and the minimum luminance of the eye chart is Lmin, a contrast C of the eye chart is expressed by the following Expression (1), for example.

[Formula 1]

$$C = \frac{Lmax - Lmin}{Lmax} \quad \cdots (1)$$

In a case of an eye chart with black letters or the like drawn on a white background (hereinafter, sometimes referred to as a black-and-white eye chart), assuming that luminance of the black portion (minimum luminance Lmin) is 0, the contrast C of the eye chart will be 1 from Expression (1). In a case of an eye chart with gray letters or the like drawn on a white background, assuming that luminance of the white portion (maximum luminance Lmax) is 200 and luminance of the gray portion (minimum luminance Lmin) is 50, the contrast C of the eye chart will be 0.75 from Expression (1). Furthermore, the luminance is expressed as a gradation value of 256 gradations as an example. The minimum contrast value of the eye chart visible from a wearer is referred to as a contrast threshold. The contrast sensitivity is a reciprocal of the contrast threshold. For example, when the contrast threshold is 0.1, the contrast sensitivity is 10. The contrast sensitivity also varies depending on the spatial frequency of the eye chart. In general, when the spatial frequency of the eye chart is 8 [cycle/deg] or more, the lower the spatial frequency, the higher the contrast sensitivity. It is also known that the contrast sensitivity to a specific spatial frequency and the contrast sensitivity to a plurality of spatial frequencies are different from each other depending on individual differences.

Even in the eye chart with the same size, the spatial frequency becomes lower as the distance to the eye chart becomes closer. When the wearer of the eyeglass lens has a higher contrast sensitivity to the eye chart with a low spatial frequency compared to the contrast sensitivity to the eye chart with a high spatial frequency, and can clearly view a closer object (object to be viewed) through the eyeglass lens, the contrast sensitivity to the object to be viewed increases, and thus higher visibility can be obtained. In the eyeglass lens called the progressive-power lens, the addition power at a predetermined position in the progressive portion between the distance portion and the near portion is designed to be higher than the standard setting addition power. With such a design, a closer object to be viewed can be clearly viewed through the progressive portion of the eyeglass lens, and the contrast sensitivity to the object to be viewed increases, whereby higher visibility can be obtained. Therefore, it is possible to design an appropriate eyeglass lens by a change in the distribution of addition power in the progressive portion of the eyeglass lens depending on the change in the contrast sensitivity of the wearer corresponding to the change in the spatial frequency of the object to be viewed.

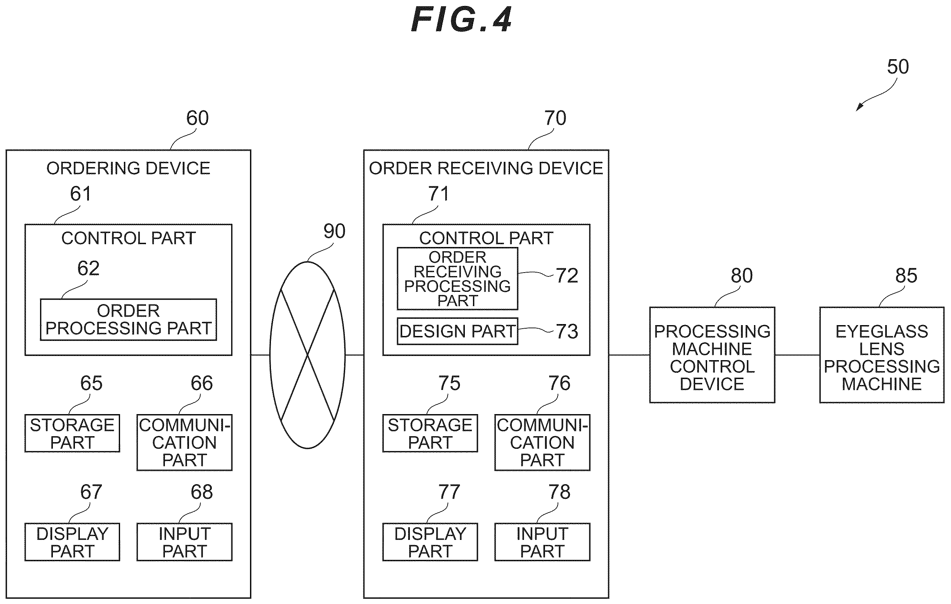

An eyeglass lens ordering and order receiving system for producing a pair of eyeglass lenses 1 according to the present embodiment will be described below. FIG. 4 shows an eyeglass lens ordering and order receiving system 50. As shown in FIG. 4, the eyeglass lens ordering and order receiving system 50 comprises an ordering device 60 installed in an optician's store (orderer's side), an order receiving device 70 installed in a lens manufacturer, a processing machine control device 80, and an eyeglass lens processing machine 85. The ordering device 60 and the order receiving device 70 are communicably connected to each other via a network 90 such as the Internet. The order receiving device 70 is connected with the processing machine control device 80, and the processing machine control device 80 is connected with the eyeglass lens processing machine 85. Note that only one ordering device 60 is shown in FIG. 4 for convenience of illustration, but actually a plurality of ordering devices 60 installed in a plurality of optician's stores is connected to the order receiving device 70.

The ordering device 60 is a computer that performs order processing for the eyeglass lens 10. The ordering device 60 comprises a control part 61, a storage part 65, a communication part 66, a display part 67, and an input part 68. The control part 61 executes a program stored in the storage part 65 and thereby controls the ordering device 60. The control part 61 has an order processing part 62 that performs order processing for the eyeglass lens 10. The communication part 66 communicates with the order receiving device 70 via the network 90. The display part 67 is configured using a display device such as a CRT and an LCD display. The display part 67 displays an order screen for inputting information (ordering information) on the eyeglass lenses to be ordered. The input part 68 is configured using, for example, a mouse and a keyboard. For example, ordering information according to the contents displayed on the order screen is input via the input part 68. Note that the display part 67 and the input part 68 may be configured integrally using a touch panel or the like.

The order receiving device 70 is a computer that performs order receiving processing, design processing, and arithmetic processing of the optical performance of the eyeglass lens 10. The order receiving device 70 comprises a control part 71, a storage part 75, a communication part 76, a display part 77, and an input part 78. The control part 71 executes a program stored in the storage part 75 and thereby controls the order receiving device 70. The control part 71 includes an order receiving processing part 72 that performs order processing of the eyeglass lens 10, and a design part 73 that performs design processing of the eyeglass lens 10. The storage part 75 readably stores various data for designing eyeglass lenses. The communication part 76 communicates with the ordering device 60 via the network 90. The communication part 76 also communicates with the processing machine control device 80. The display part 77 is configured using a display device such as a CRT and an LCD display. The display part 77 displays a design result of the eyeglass lens 10, and the like. The input part 78 is configured using, for example, a mouse and a keyboard. Note that the display part 77 and the input part 78 may be configured integrally using a touch panel or the like.

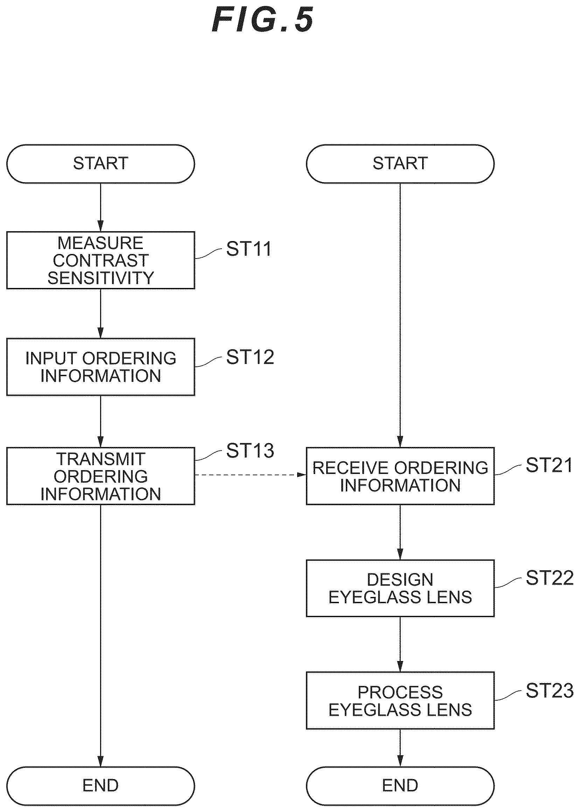

A procedure for producing and providing a pair of eyeglass lenses 1 using the eyeglass lens ordering and order receiving system 50 will be described below with reference to FIG. 5. FIG. 5 is a flowchart showing a flow of producing and providing the pair of eyeglass lenses 1. A left side of FIG. 5 shows a procedure performed on the optician's store side, and a right side of FIG. 5 shows a procedure performed on the lens manufacturer side. An orderer measures contrast sensitivity of a wearer of the eyeglass lens (step ST11). A measurement method of the contrast sensitivity will be described in detail below.

Next, the orderer causes the display part 67 of the ordering device 60 to display the order screen, and inputs ordering information via the input part 68 (step ST12). At this time, the orderer determines ordering information for the eyeglass lenses to be ordered that includes information on contrast sensitivity of the wearer acquired when measuring the contrast sensitivity of the wearer.

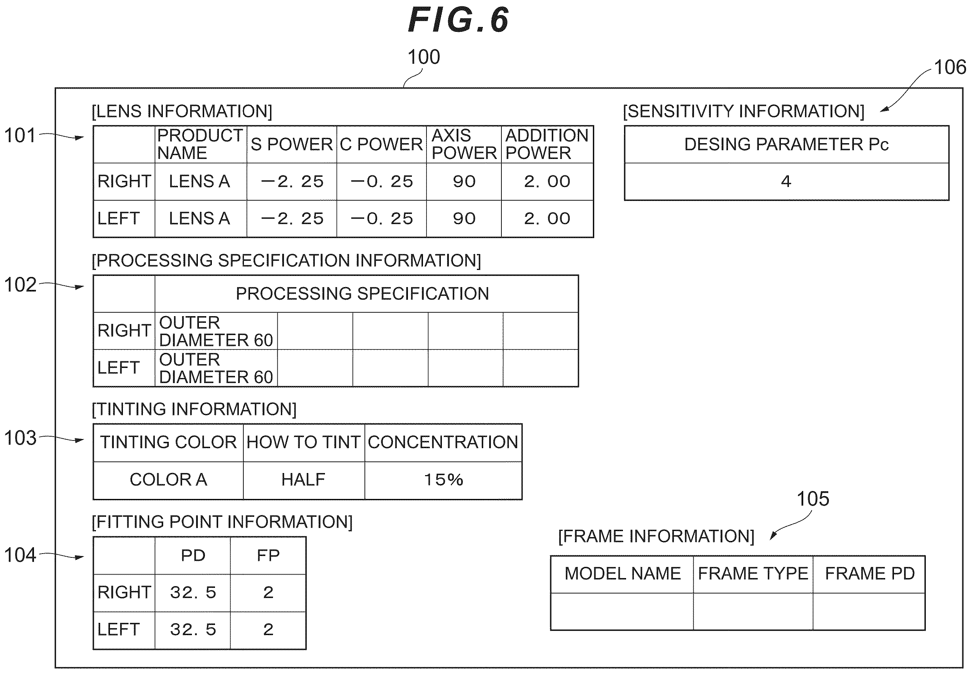

FIG. 6 shows an example of the order screen 100. A lens information item 101 is input with a product name of the lens to be ordered, and items related to the lens power to be ordered, including spherical power (S power), astigmatism power (C power), astigmatism axis power, and addition power. A processing specification information item 102 is used for specifying the outer diameter and arbitrary point thickness of the lens to be ordered. A tinting information item 103 is used for specifying a color of the lens. Fitting point (FP) information 104 is used to input positional information of eyes of the wearer. PD represents an interpupillary distance. A frame information item 105 is input with a frame model name, a frame type, and the like. A sensitivity information item 106 is input with numerical values representing a design parameter Pc obtained based on the measurement result of the contrast sensitivity of the wearer in previous step S11, as information regarding the contrast sensitivity of the wearer. In the example shown in FIG. 6, the design parameter Pc is expressed by a numerical value on a scale of one to 10 (as a specific example, a case is expressed where the design parameter Pc is "4").

The design parameter Pc will be described in detail below. The design parameter Pc may be set to an integer value from "-5" to "5", or may be set to an integer value from "0" to "10". The design parameter Pc is not limited to a numerical value, and may be classified using labels of "A", "B", "C", and the like. Furthermore, the sensitivity information item 106 may be input with numerical values indicating design parameters Pc respectively corresponding to the right eye and the left eye. The sensitivity information item 106 may be input, as the information regarding the contrast sensitivity of the wearer, with not only the design parameter Pc, but also a numerical value indicating the contrast sensitivity of the wearer acquired during the measurement of the contrast sensitivity of the wearer, that is, information regarding the measurement result of the contrast of the wearer.

When the orderer inputs into each item on the order screen 100 shown in FIG. 6 and clicks a send button (not shown), the order processing part 62 of the ordering device 60 acquires information (ordering information) input into each item on the order screen 100. The ordering device 60 sends the ordering information acquired by the order processing part 62 to the order receiving device 70 via the communication part 66 (step ST13). In the ordering device 60, the process of displaying the order screen 100, the process of acquiring the ordering information input through the order screen 100, and the process of transmitting the ordering information to the order receiving device 70 are performed by the control part 61 of the ordering device 60 executing a predetermined program installed in advance in the storage part 65.

When the ordering information is transmitted from the ordering device 60, the order receiving processing part 72 of the order receiving device 70 receives the ordering information transmitted from the ordering device 60 via the communication part 76 (step ST21). The design part 73 of the order receiving device 70 designs the eyeglass lens 10 based on the received ordering information (step ST22). The design method of the eyeglass lens 10 will be described in detail below. The order receiving device 70 outputs design data of the eyeglass lens 10 designed by the design part 73 to the processing machine control device 80 via the communication part 76.

Based on the design data output from the order receiving device 70, the processing machine control device 80 sends processing instructions to the eyeglass lens processing machine 85 (step ST23). As a result, the eyeglass lens processing machine 85 processes and produces the eyeglass lens based on the design data. The eyeglass lens 10 (i.e., the pair of eyeglass lenses 1) produced by the eyeglass lens processing machine 85 is shipped to the optician's store, is fitted into the eyeglass frame, and is provided to a customer (the wearer). In the order receiving device 70, the process of receiving the ordering information from the ordering device 60, the process of designing the eyeglass lens based on the received ordering information, and the process of outputting the design data of the eyeglass lens to the processing machine control device 80 are performed by the control part 71 of the order receiving device 70 executing a predetermined program installed in advance in the storage part 75.

Next, a method of measuring contrast sensitivity will be described. In the present embodiment, at least two eye charts having different spatial frequencies are used to measure contrast sensitivity of a wearer of an eyeglass lens, and the measurement result of the contrast sensitivity is used for a design of the eyeglass lens. For example, letters, pictures, Landolt rings, and Gabor patches are used as the eye chart. Furthermore, the wearer views the eye chart at a predetermined distance, and determines the presence or absence of the eye chart, an orientation of the eye chart, and letters or pictures constituting the eye chart, whereby the contrast sensitivity can be measured. The predetermined distance is, for example, a distance, at which the contrast sensitivity can be measured, from the eyeball of the wearer to the eye chart. The spatial frequency of the eye chart used in the measurement of the contrast sensitivity is a spatial frequency when the eye chart is viewed at a predetermined distance (a fixed distance).

At least two kinds of prescribed spatial frequencies may be used as the spatial frequency of the eye chart used in the measurement of the contrast sensitivity. Alternatively, a black-and-white eye chart (that is, an eye chart with a contrast of 1) may be used first to obtain the minimum eye chart that the wearer can view, and the spatial frequency of the eye chart used in the measurement of the contrast sensitivity may be set based on the size of the eye chart. Since the spatial frequency of the eye chart used in the measurement of the contrast sensitivity is set based on the size of the minimum eye chart visible to the wearer, it is possible to measure the contrast sensitivity with higher accuracy using at least two eye charts having different spatial frequencies set according to visual acuity of the wearer.

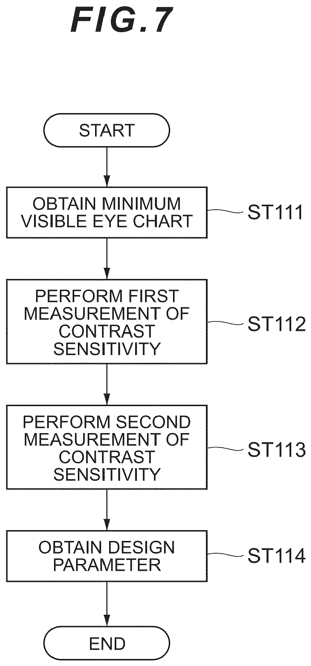

FIG. 7 is a flowchart showing a procedure for measuring the contrast sensitivity of the wearer corresponding to step ST11. The procedure shown in FIG. 7 represents a procedure for setting the spatial frequency of the eye chart used in the measurement of the contrast sensitivity based on the size of the minimum eye chart visible to the wearer. When the contrast sensitivity of the wearer is measured, first, an orderer causes the wearer to view a black-and-white eye chart (an eye chart with a contrast of 1) at a predetermined distance with both eyes, and obtains a minimum eye chart visible to the wearer (step ST111). In addition, the orderer may cause the wearer to view the black-and-white eye chart at a predetermined distance with one eye (at least one of the left and right eyes), and obtain a minimum eye chart visible to the wearer. The minimum eye chart visible to the wearer may be an eye chart corresponding to corrected visual acuity of the wearer, or an eye chart corresponding to uncorrected visual acuity of the wearer.

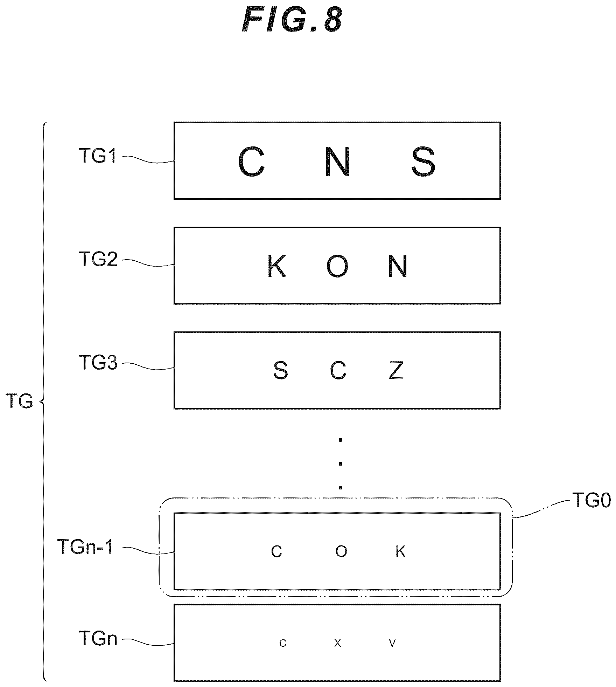

In the present embodiment, the black-and-white eye chart (the eye chart with the contrast of 1) is referred to as a reference eye chart. FIG. 8 shows an example of a reference eye chart. In the example shown in FIG. 8, letters such as alphabets are used as a reference eye chart TG. As described above, as the reference eye chart TG, pictures, Landolt rings, and Gabor patches may be used without being limited to the letters. In the present embodiment, a plurality of reference eye charts TG having different spatial frequencies may be referred to as reference eye charts TG1 to TGn (n being an integer of 2 or more). For example, when n = 3, the reference eye charts TG1 to TG3 indicate the reference eye chart TG1 with the lowest spatial frequency, the reference eye chart TG2 with the second lowest spatial frequency, and the reference eye chart TG3 with the highest spatial frequency. Similarly, the reference eye charts TG1 to TGn indicate the reference eye chart TG1 with the lowest spatial frequency, ..., and the reference eye chart TGn with the highest spatial frequency. Note that the spatial frequencies of the plurality of reference eye charts TG (reference eye charts TG1 to TGn) are the spatial frequencies when each of the reference eye charts TG is viewed at a predetermined distance (a fixed distance). As the size of the reference eye chart TG becomes smaller, the spatial frequency of the reference eye chart TG becomes higher.

In the example shown in FIG. 8, the wearer is allowed to sequentially view the reference eye charts TG1 to TGn, and select the reference eye charts, which are visible, from the reference eye charts TG1 to TGn. Then, out of the reference eye charts selected by the wearer, the minimum reference eye chart TG0 visible to the wearer is obtained. At this time, a spatial frequency of the minimum reference eye chart TG0 visible to the wearer is obtained. In FIG. 8, a two-dot chain line indicates that the reference eye chart TGn-1 having the (n-1)-th lowest spatial frequency (in other words, the second highest spatial frequency) is obtained as the minimum reference eye chart TG0 visible to the wearer. Hereinafter, the spatial frequency of the minimum reference eye chart TG0 visible to the wearer may be referred to as a reference spatial frequency f0. In this case, a first spatial frequency f1 used in a first measurement of the contrast sensitivity is calculated by multiplying the reference spatial frequency f0 by a coefficient $\alpha$ ($\alpha$ = 0.3 to 0.75). In addition, a second spatial frequency f2 used in a second measurement of the contrast sensitivity is calculated by multiplying the first spatial frequency f1 by a coefficient $\beta$ ($\beta$ = 0.3 to 0.75). Note that f1 = $\alpha \times$ f0, and f2 = $\beta \times$ f1.

The contrast sensitivity varies depending on the spatial frequency of the eye chart. It is known that, under general conditions, the contrast sensitivity is maximized when the spatial frequency of the eye chart is approximately 2 to 8 [cycle/deg]. In the present embodiment, using an eye chart having a spatial frequency lower than the spatial frequency corresponding to the visual acuity of the wearer and higher than the spatial frequency at which the contrast sensitivity is maximized, the first measurement and the second measurement of the contrast sensitivity are performed. Therefore, the coefficient $\alpha$ and the coefficient $\beta$ are adjusted such that the second spatial frequency f2 (and the first spatial frequency f1) is higher than 8 [cycle/deg].

In addition, instead of the reference spatial frequency f0 of the reference eye chart TG0, the visual acuity corresponding to the reference eye chart TG0 may be calculated as an expression representing the size of the eye chart. Hereinafter, the visual acuity corresponding to the reference eye chart TG0 may be referred to as reference visual acuity VA0. In this case, the orderer multiplies the reference visual acuity VA0 by the coefficient $\alpha$ described above to calculate first visual acuity VA1 used to select a first eye chart which will be described below. Furthermore, the orderer multiplies the first visual acuity VA1 by the coefficient $\beta$ described above to calculate second visual acuity VA2 used to select $\alpha$ second eye chart which will be described below. Note that VA1 = $\alpha \times$ VA0, and VA2 = $\beta \times$ VA1. The reference visual acuity VA0, the first visual acuity VA1, and the second visual acuity VA2 are expressed using decimal visual acuity. The reference visual acuity VA0, the first visual acuity VA1, and the second visual acuity VA2 may be expressed using fractional visual acuity or Log Mar (Logarithm of the Minimum angle of resolution) without being limited to the decimal visual acuity.

Next, the orderer performs the first measurement of the contrast sensitivity by causing the wearer to view the eye chart having the first spatial frequency f1 (or corresponding to the first visual acuity VA1) at a predetermined distance with both eyes (step ST112). Alternatively, the orderer may perform the first measurement of the contrast sensitivity by causing the wearer to view the eye chart having the first spatial frequency f1 at a predetermined distance with one eye (at least one of the left eye and the right eye). In addition, when the first measurement of the contrast sensitivity is performed, the first spatial frequency f1 described in the previous step ST111 may be calculated.

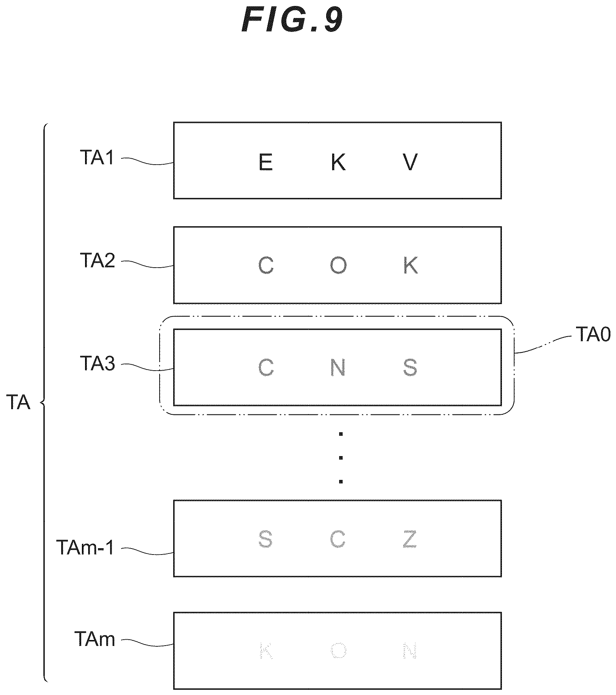

In the present embodiment, the eye chart having the first spatial frequency f1 (or corresponding to the first visual acuity VA1) is referred to as a first eye chart. FIG. 9 shows an example of the first eye chart. In the example shown in FIG. 9, similarly to the reference eye chart TG, letters such as alphabets are used as the first eye chart TA. As described above, as the first eye chart TA, pictures, Landolt rings, and Gabor patches may be used without being limited to the letters. In the present embodiment, a plurality of first eye charts TA having different contrasts from each other may be referred to as first eye charts TA1 to TAm (m being an integer of 2 or more). For example, when m = 3, the first eye charts TA1 to TA3 indicate the first eye chart TA1 with the highest contrast, the first eye chart TA2 with the second highest contrast, and the first eye chart TA3 with the lowest contrast. Similarly, the first eye charts TA1 to TAm indicate the first eye chart TA1 with the highest contrast, ..., and the first eye chart TAm with the lowest contrast.

In the example shown in FIG. 9, the wearer is allowed to sequentially view the first eye charts TA1 to TAm, and select the first eye charts, which are visible, from the first eye charts TA1 to TAm. Then, out of the first eye charts selected by the wearer, the first eye chart TA0 visible to the wearer with the minimum contrast is obtained. At this time, based on the contrast of the first eye chart TA0 visible to the wearer with the minimum contrast, the contrast sensitivity is obtained which is the measurement result of the first measurement. In FIG. 9, a two-dot chain line indicates that the first eye chart TA3 having the third highest contrast is obtained as the first eye chart TA0 visible to the wearer with the minimum contrast. Hereinafter, the contrast sensitivity based on the contrast of the first eye chart TA0 visible to the wearer with the minimum contrast, that is, the contrast sensitivity measured using the first eye charts TA1 to TAm, may be referred to as first contrast sensitivity CSF1.

Next, the orderer performs the second measurement of the contrast sensitivity by causing the wearer to view the eye chart having the second spatial frequency f2 (or corresponding to the second visual acuity VA2) at a predetermined distance with both eyes (step ST113). Alternatively, the orderer may perform the second measurement of the contrast sensitivity by causing the wearer to view the eye chart having the second spatial frequency f2 at a predetermined distance with one eye (at least one of the left eye and the right eye). In addition, when the second measurement of the contrast sensitivity is performed, the second spatial frequency f2 described in the previous step ST111 may be calculated.

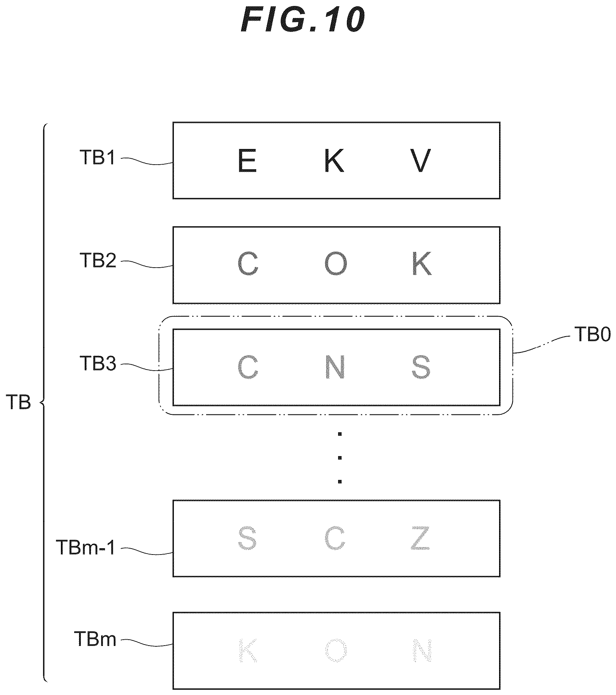

In the present embodiment, the eye chart having the second spatial frequency f2 (or corresponding to the second visual acuity VA2) lower than the first spatial frequency f1 is referred to as a second eye chart. FIG. 10 shows an example of the second eye chart. In the example shown in FIG. 10, similarly to the reference eye chart TG, letters such as alphabets are used as the second eye chart TB. As described above, as the second eye chart TB, pictures, Landolt rings, and Gabor patches may be used without being limited to the letters. In the present embodiment, a plurality of second eye charts TB having different contrasts from each other may be referred to as second eye charts TB1 to TBm. For example, when m = 3, the second eye charts TB1 to TB3 indicate the second eye chart TB1 with the highest contrast, the second eye chart TB2 with the second highest contrast, and the second eye chart TB3 with the lowest contrast. Similarly, the second eye charts TB1 to TBm indicate the second eye chart TB1 with the highest contrast, ..., and the second eye chart TBm with the lowest contrast.

In the example shown in FIG. 10, the wearer is allowed to sequentially view the second eye charts TB1 to TBm, and select the second eye charts, which are visible, from the second eye charts TB1 to TBm. Then, out of the second eye charts selected by the wearer, the second eye chart TB0 visible to the wearer with the minimum contrast is obtained. At this time, based on the contrast of the second eye chart TB0 visible to the wearer with the minimum contrast, the contrast sensitivity is obtained which is the measurement result of the second measurement. In FIG. 10, a two-dot chain line indicates that the second eye chart TB2 having the second highest contrast is obtained as the second eye chart TB0 visible to the wearer with the minimum contrast. Hereinafter, the contrast sensitivity based on the contrast of the second eye chart TB0 visible to the wearer with the minimum contrast, that is, the contrast sensitivity measured using the second eye charts TB1 to TBm, may be referred to as second contrast sensitivity CSF2.

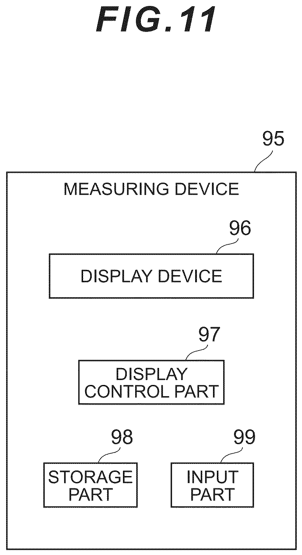

Next, the orderer uses a measuring device 95 (see FIG. 11) to be described below to obtain a design parameter Pc based on the first contrast sensitivity CSF1 and the second contrast sensitivity CSF2 measured in the first measurement and the second measurement of the contrast sensitivity (step ST 114). After the design parameter Pc is obtained, the process proceeds to a step of inputting ordering information (step ST 12).

In the present embodiment, a display device 96 (see FIG. 11) provided in the measuring device 95 is used to allow the wearer to view an image of the reference eye chart TG, an image of the first eye chart TA, or an image of the second eye chart TB. The measuring device 95 may be configured, for example, using a tablet PC (Personal Computer), or may be configured using a notebook PC. The measuring device 95 may be incorporated in the ordering device 60, or may be installed at an optician's store (orderer's side) separately from the ordering device 60. The measuring device 95 includes a display device 96, a display control part 97, a storage part 98, and an input part 99, as shown schematically in FIG. 11. The display device 96 is configured using a liquid crystal display, for example. When the contrast sensitivity is measured, the distance between the eyeball of the wearer and the display device 96 is kept at a predetermined distance.

The display control part 97 selects any one of the reference eye chart TG, the first eye chart TA, and the second eye chart TB to control the display device 96 to display the one. The display control part 97 controls the display device 96 to display at least one of the plurality of reference eye charts TG (reference eye charts TG1 to TGn). The display control part 97 controls the display device 96 to display at least one of the plurality of first eye charts TA (first eye charts TA1 to TAm). The display control part 97 controls the display device 96 to display at least one of the plurality of second eye charts TB (second eye charts TB1 to TBm). The storage part 98 readably stores image data of the reference eye chart TG, the first eye chart TA, and the second eye chart TB that are generated in advance.

The input part 99 is configured using, for example, a touch panel and a keyboard. For example, an input operation is performed on the input part 99 to select a visible one from the reference eye charts TG1 to TGn. Furthermore, an input operation is performed on the input part 99 to select a visible one from the first eye charts TA1 to TAm. An input operation is performed on the input part 99 to select a visible one from the second eye charts TB1 to TBm.

The display control part 97 may perform control to display the reference eye charts TG1 to TGn one by one sequentially from the lowest spatial frequency, or may perform control to display the reference eye charts TG1 to TGn one by one in random order. The display control part 97 may perform control, when the wearer can view the reference eye chart displayed on the display device 96, to display a reference eye chart with a higher spatial frequency than the above reference eye chart, and may perform control, when the wearer cannot view the reference eye chart displayed on the display device 96, to display a reference eye chart with a lower spatial frequency than the above reference eye chart.

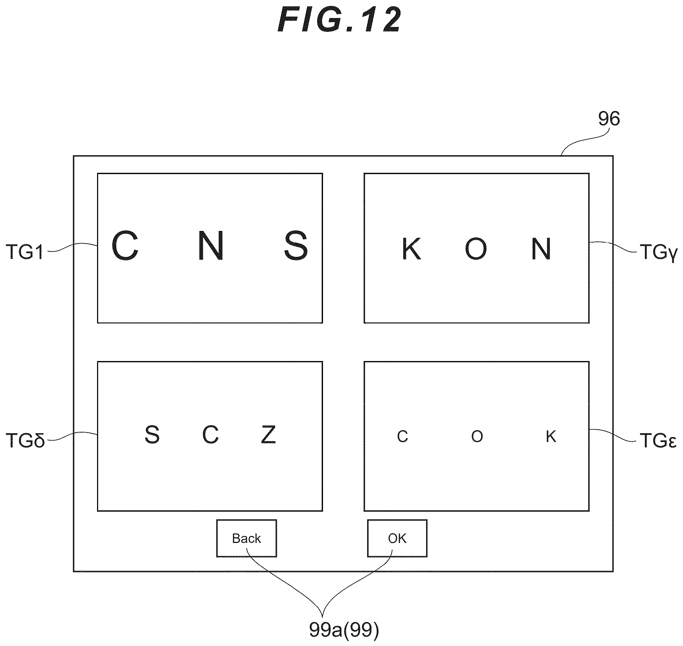

In addition, the display control part 97 may perform control to simultaneously display some (for example, four) of the reference eye charts TG1 to TGn, for example, as shown in FIG. 12. Specifically, control may be performed to display, on a screen of the display device 96, the reference eye chart TG1 with the lowest spatial frequency, the reference eye chart TG$\gamma$ with a higher spatial frequency than the reference eye chart TG1, the reference eye chart TG$\delta$ with a higher spatial frequency than the reference eye chart TGy, and the reference eye chart TG$\varepsilon$ with a higher spatial frequency than the reference eye chart TG$\delta$. In addition, when an operation is performed to select any one of these reference eye charts TG1, TGy, TG$\delta$, and TG$\varepsilon$, control may be performed to display a plurality of reference eye charts (for example, four) with a spatial frequency close to the spatial frequency of the selected reference eye chart, and an operation may be performed to select it as the minimum reference eye chart TG0 visible to the wearer.

The display control part 97 may perform control to display the first eye charts TA1 to TAm one by one sequentially from the highest contrast, or may perform control to display the first eye charts TA1 to TAm one by one in random order. The display control part 97 may perform control, when the wearer can view the first eye chart displayed on the display device 96, to display a first eye chart with a lower contrast than the above first eye chart, and may perform control, when the wearer cannot view the first eye chart displayed on the display device 96, to display a first eye chart with a higher contrast than the above first eye chart.

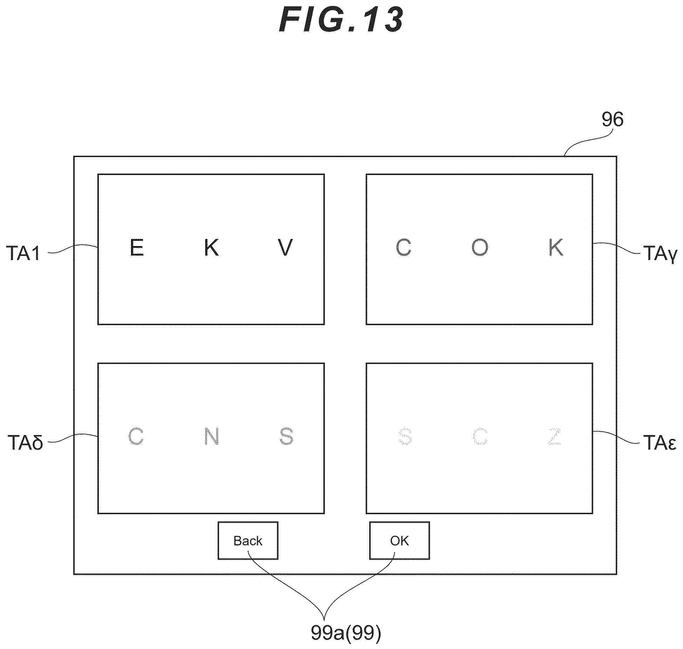

In addition, the display control part 97 may perform control to simultaneously display some (for example, four) of the first eye charts TA1 to TAm, for example, as shown in FIG. 13. Specifically, control may be performed to display, on the screen of the display device 96, the first eye chart TA1 with the highest contrast, the first eye chart TA$\gamma$ with a lower contrast than the first eye chart TA1, the first eye chart TA$\delta$ with a lower contrast than the first eye chart TAy, and the first eye chart TA$\epsilon$ with a lower contrast than the first eye chart TA$\delta$. In addition, when an operation is performed to select any one of these first eye charts TA1, TAy, TA$\delta$, and TA$\epsilon$, control may be performed to display a plurality of first eye charts (for example, four) with a contrast close to the contrast of the selected first eye chart, and an operation may be performed to select it as the first eye chart TA0 visible to the wearer with the minimum contrast.

The display control part 97 may perform control to display the second eye charts TB1 to TBm one by one sequentially from the highest contrast, or may perform control to display the second eye charts TB1 to TBm one by one in random order. The display control part 97 may perform control, when the wearer can view the second eye chart displayed on the display device 96, to display a second eye chart with a lower contrast than the above second eye chart, and may perform control, when the wearer cannot view the second eye chart displayed on the display device 96, to display a second eye chart with a higher contrast than the above second eye chart.

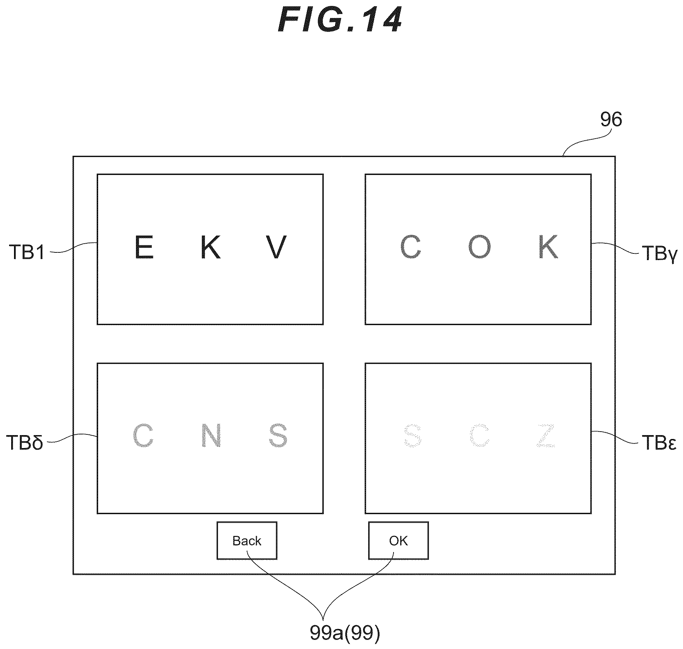

In addition, the display control part 97 may perform control to simultaneously display some (for example, four) of the second eye charts TB1 to TBm, for example, as shown in FIG. 14. Specifically, control may be performed to display, on the screen of the display device 96, the second eye chart TB1 with the highest contrast, the second eye chart TB$\gamma$ with a lower contrast than the second eye chart TB1, the second eye chart TB$\delta$ with a lower contrast than the second eye chart TBy, and the second eye chart TB$\epsilon$ with a lower contrast than the second eye chart TB$\delta$. In addition, when an operation is performed to select any one of these second eye charts TB1, TBy, TB$\delta$, and TB$\epsilon$, control may be performed to display a plurality of second eye charts (for example, four) with a contrast close to the contrast of the selected second eye chart, and an operation may be performed to select it as the second eye chart TB0 visible to the wearer with the minimum contrast.

In the examples shown in FIGS. 12 to 14, an image of an input instruction part 99a is displayed on the screen of the display device 96 as the input part 99, and is tapped or clicked to switch the image displayed on the display device 96.

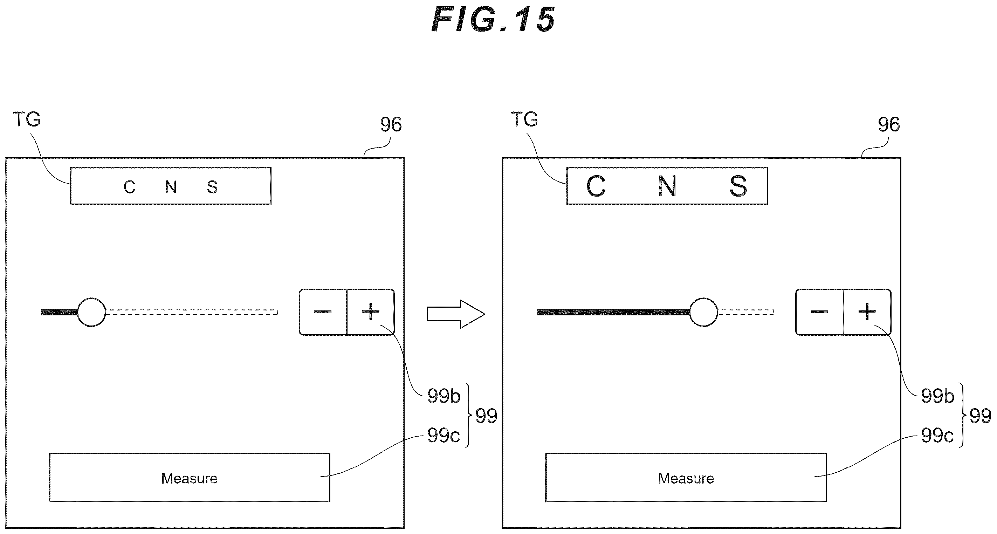

The display control part 97 may perform control to display the reference eye chart TG on the screen of the display device 96 such that the spatial frequency (magnitude) of the reference eye chart TG can be changed continuously (or stepwise), for example, as shown in FIG. 15. In the example shown in FIG. 15, an image of a first input instruction part 99b and an image of a second input instruction part 99c are displayed, as the input part 99, on the screen of the display device 96, the first input instruction part 99b being tapped or clicked to change the spatial frequency (magnitude) of the reference eye chart TG, the second input instruction part 99c being tapped or clicked to determine as the minimum reference eye chart TG0 visible to the wearer.

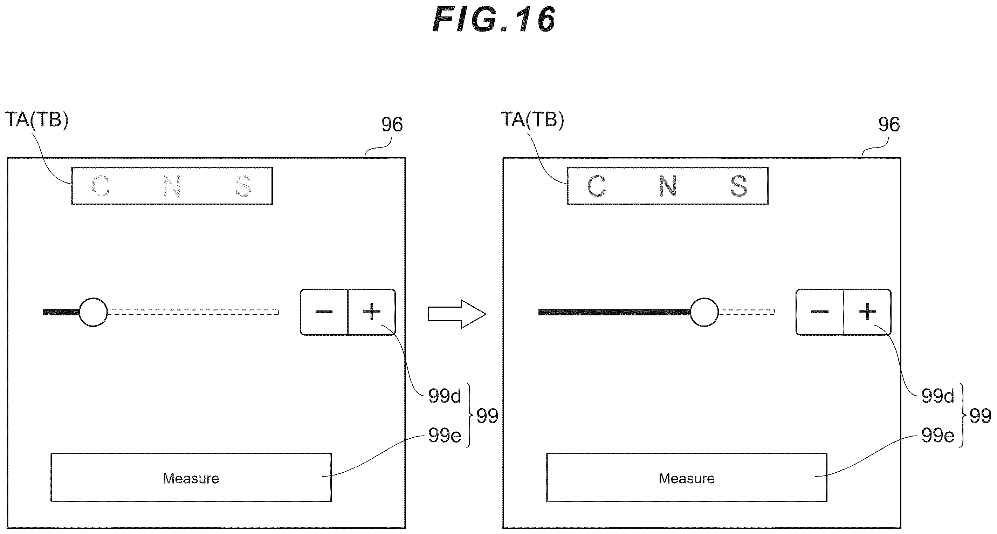

The display control part 97 may perform control to display the first eye chart TA on the screen of the display device 96 such that the contrast of the first eye chart TA can be changed continuously (or stepwise), for example, as shown in FIG. 16. Similarly, the display control part 97 may perform control to display the second eye chart TB on the screen of the display device 96 such that the contrast of the second eye chart TB can be changed continuously (or stepwise). In the example shown in FIG. 16, an image of a first input instruction part 99d and an image of a second input instruction part 99e are displayed, as the input part 99, on the screen of the display device 96, the first input instruction part 99d being tapped or clicked to change the contrast of the first eye chart TA (the second eye chart TB), the second input instruction part 99e being tapped or clicked to determine as the first eye chart TA0 (the second eye chart TBO) having the minimum contrast visible to the wearer.

Furthermore, spatial frequency dependency of the contrast sensitivity, that is, contrast sensitivity CFS(f) approximated by an exponential function of the spatial frequency, is obtained based on the first contrast sensitivity CSF1 and the second contrast sensitivity CSF2. The contrast sensitivity CSF(f) is approximated by the following Expression (2).

[Formula 2]

$$CSF(f) = A \times exp(-B \times f) \qquad ...(2)$$

Where, "f" represents the spatial frequency of the eye chart when viewed at a predetermined distance (fixed distance). "A" represents an arbitrary coefficient. "B" represents a parameter that indicates how much the contrast sensitivity changes with respect to the change in the spatial frequency of the eye chart. Hereinafter, such a parameter is referred to as a measurement parameter B. If an expression where f = f1 and CSF(f) = CSF1 and an expression where f = f2 and CSF(f) = CSF2 are put into Expression (2), the measurement parameter B can be expressed by the following Expression (3).

[Formula 3]

$$B = -\frac{\log_e CSF2 - \log_e CSF1}{f2 - f1} \quad \cdots (3)$$

Using Expression (3), the measurement parameter B can be obtained based on the first spatial frequency f1 and the second spatial frequency f2, and the first contrast sensitivity CSF1 and the second contrast sensitivity CSF2. In Expression (3), the first spatial frequency f1 and the second spatial frequency f2 may be replaced with the first visual acuity VA1 and the second visual acuity VA2. Thus, the measurement parameter B can be obtained based on the first visual acuity VA1 and the second visual acuity VA2, and the first contrast sensitivity CSF1 and the second contrast sensitivity CSF2.

The measurement parameter B obtained using Expression (3) is converted into a design parameter Pc. The design parameter Pc is a simple numerical parameter that is set based on the change in contrast sensitivity (that is, the measurement parameter B) measured in advance for a plurality of wearers and is used in the design of the eyeglass lens. For example, the design parameter Pc may be set to an integer value from "- 5" to "5", with 0 being the standard setting based on an average value of the change in contrast sensitivity measured in advance for a plurality of wearers (that is, an average value of the measurement parameter B). The design parameter Pc may be set to an integer value from "0" to "10" depending on the range of the measurement parameter B that can be measured in actual human eyesight in the relationship between the spatial frequency and the contrast sensitivity approximated by Expression (2). The measurement parameter B and the design parameter Pc may be calculated by a calculation part (not shown) of the measuring device 95. When the measurement parameter B is converted into the design parameter Pc, the value of the coefficient A may be added during the calculation. In the present embodiment, that the value of the design parameter Pc is large means that the change in the contrast sensitivity is large relative to the change in the spatial frequency of the eye chart.

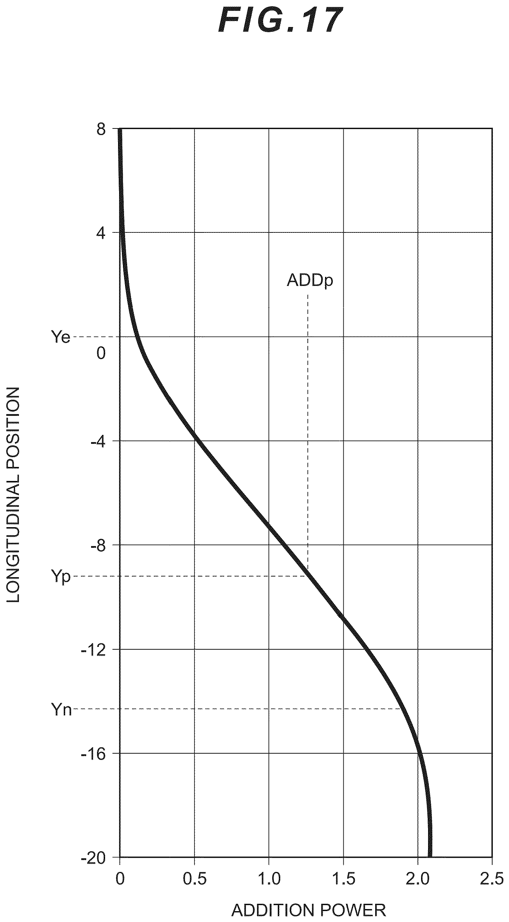

Next, a method of designing the eyeglass lens 10 will be described. In the present embodiment, the design of the eyeglass lens 10 is optimized using the design parameter Pc based on the contrast sensitivity of the wearer. Specifically, as shown in FIG. 17, a position (longitudinal position) Yp is set at which the value of the addition power is changed on the eyeglass lens 10. A vertical axis of the graph in FIG. 17 indicates the above-described longitudinal position [mm] on the eyeglass lens 10. A horizontal axis of the graph in FIG. 17 indicates an addition power [D] at each longitudinal position on the eyeglass lens 10. Hereinafter, the position Yp, at which the value of the additional power is changed on the eyeglass lens 10, may be referred to as an addition power change position Yp.

The addition power change position Yp is set at a position between a position (longitudinal position) Yn at which a near prescribed power (addition power) is provided on the eyeglass lens 10 and a position (longitudinal position) Ye through which the line of sight passes on the eyeglass lens 10 when wearing eyeglasses and looking horizontally. The position Yn at which the near prescribed power (addition power) is provided on the eyeglass lens 10 may be a position (longitudinal position) of a near reference point in the eyeglass lens 10. The position Ye through which the line of sight passes on the eyeglass lens 10 when wearing the eyeglasses and looking horizontally may be set to a coordinate position of the origin, or may be a position (longitudinal position) of the optical center of the eyeglass lens 10.

The addition power change position Yp may be a coordinate position of $0.5 \times$ Yn to $0.9 \times$ Yn. Thus, the addition power at the addition power change position Yp in the eyeglass lens 10 (progressive portion) can be set to be higher, and the object (object to be viewed) appears closer and larger through the progressive portion of the eyeglass lens 10, whereby higher visibility can be obtained.

Then, a standard setting addition power ADDp0 at the addition power change position Yp is changed according to the value of the design parameter Pc based on the contrast sensitivity of the wearer to obtain an addition power ADDp at the addition power change position Yp. The addition power ADDp at the addition power change position Yp is set to be smaller than the addition power corresponding to the near prescribed power (at the position Yn) in a range of $0.8 \times$ ADDp0 to $1.2 \times$ ADDp0.

As described above, the design parameter Pc may be set to the integer value from "- 5" to "5", or may be set to the integer value from "0" to "10". When the design parameter Pc is set to the integer value from "- 5" to "5", the addition power ADDp at the addition power change position Yp is expressed by the following Expression (4).

[Formula 4]

$$ADDp = ADDp0 + k \times Pc \cdots (4)$$

Where, k indicates a predetermined coefficient that takes a value of 0.1 to 0.4, and can be changed depending on design conditions and a prescribed power for each eyeglass lens product.

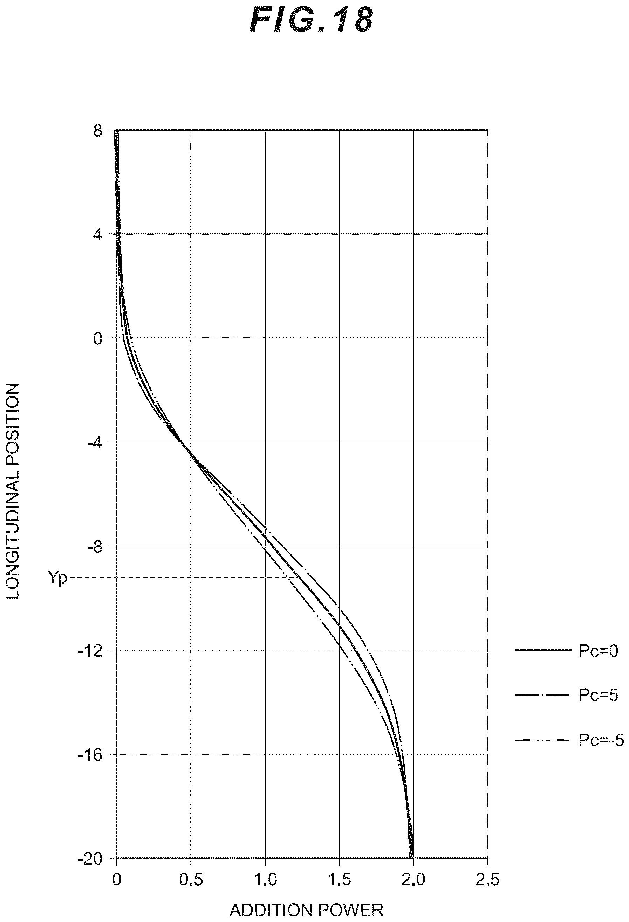

FIG. 18 shows an example in which the addition power at the addition power change position Yp is changed using the design parameter Pc set to the integer value from "- 5" to "5". A vertical axis of the graph in FIG. 18 indicates the above-described longitudinal position [mm] on the eyeglass lens 10. A horizontal axis of the graph in FIG. 18 indicates the addition power [D] at each longitudinal position on the eyeglass lens 10. A solid line of the graph in FIG. 18 indicates a case where Pc = 0. A one-dot chain line of the graph in FIG. 18 indicates a case where Pc = 5. A two-dot chain line of the graph in FIG. 18 indicates a case where Pc = - 5.

It can be seen from FIG. 18 that the addition power in the progressive portion of the eyeglass lens 10 is designed to be higher overall when Pc = 5 (> 0) rather than when Pc = 0 (that is, in a case of standard setting). That the value of the design parameter Pc is large as a result of measuring the contrast sensitivity means that the change in the contrast sensitivity is large relative to the change in the spatial frequency of the object (object to be viewed). Therefore, when Pc = 5 (> 0), a closer object to be viewed can be clearly viewed through the progressive portion of the eyeglass lens 10, and the contrast sensitivity to the object to be viewed increases, whereby higher visibility can be obtained. On the other hand, it can be seen that the addition power in the progressive portion of the eyeglass lens 10 is designed to be lower overall when Pc = - 5 (< 0) rather than when Pc = 0 (that is, in a case of standard setting). That the value of the design parameter Pc is small as a result of measuring the contrast sensitivity means that the change in the contrast sensitivity is small relative to the change in the spatial frequency of the object (object to be viewed). Therefore, there is little benefit in increasing the addition power in the progressive portion of the eyeglass lens 10. Thus, the addition power in the progressive portion of the eyeglass lens 10 is designed to be lower overall when Pc = - 5 (< 0) rather than when Pc = 0, and thus the change in the addition power in the progressive portion of the eyeglass lens 10 becomes relatively gradual, whereby astigmatism in the progressive portion of the eyeglass lens 10 becomes relatively small. Therefore, the distribution of the addition power in the progressive portion of the eyeglass lens 10 is changed according to the value of the design parameter Pc obtained from the contrast sensitivity of the wearer, whereby it is possible to design an appropriate eyeglass lens according to the change in the contrast sensitivity of the wearer.

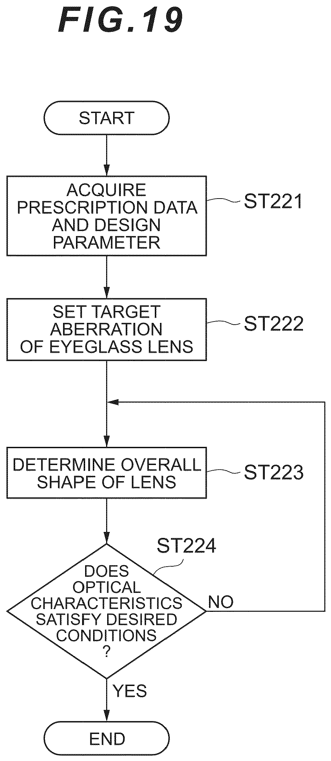

FIG. 19 is a flowchart showing a design procedure of the eyeglass lens 10 corresponding to step ST22. During the design of the eyeglass lens 10, the order receiving device 70 acquires prescription data for the eyeglass lens and the design parameter Pc based on the contrast sensitivity of the wearer (step ST221). At this time, the order receiving device 70 also acquires fitting parameters, for example, a pantoscopic angle of a frame, a wrap angle, and a distance between the eyes and the lens, as appropriate.

Next, the design part 73 of the order receiving device 70 sets a target aberration of the eyeglass lens based on the prescription data of the eyeglass lens and the design parameter Pc based on the contrast sensitivity of the wearer which are acquired previous in step ST221 (step ST222). At this time, the distribution of the addition power in the progressive portion of the eyeglass lens 10 is obtained according to the value of the design parameter Pc, using Expression (4) or the like described above.

When the target aberration of the eyeglass lens is set, the order receiving device 70 determines the overall shape of the eyeglass lens (step ST 223). After the overall shape of the eyeglass lens is determined, the order receiving device 70 determines whether optical characteristics of the eyeglass lens, for example, the refractive power and the astigmatism satisfy desired conditions (step ST 224). When the optical characteristics do not satisfy the desired conditions, that is, when it is determined in step ST224 to be NO, the process returns to previous step ST223. When the optical characteristics satisfy the desired conditions, that is, when it is determined in step ST224 to be YES, the design procedure of the eyeglass lens ends. When the respective steps (steps ST221 to ST224) for designing the eyeglass lens are completed, the process proceeds to the step (step ST23) for processing the eyeglass lens.

In the present embodiment, the contrast sensitivity of the wearer of the eyeglass lens is measured using the first eye chart TA and the second eye chart TB with different spatial frequencies from each other, and the eyeglass lens 10 is designed using the design parameter Pc based on the contrast sensitivity of the wearer. This makes it possible to change the distribution of the addition power in the progressive portion of the eyeglass lens 10 according to the value of the design parameter Pc obtained from the contrast sensitivity of the wearer. Therefore, the eyeglass lens can be appropriately designed according to the change in the contrast sensitivity of the wearer. For example, as described above, the eyeglass lens 10 is designed such that the addition power (refractive power) in the progressive portion of the eyeglass lens 10 becomes high as the difference (rate of change) in the contrast sensitivity with respect to the different spatial frequencies becomes large. This makes it possible to clearly view a closer object (object to be viewed) through the progressive portion of the eyeglass lens 10, the contrast sensitivity to the object to be viewed increases, and thus higher visibility can be obtained. In this way, the distribution of the addition power in the progressive portion of the eyeglass lens 10 is changed according to the value of the design parameter Pc obtained from the contrast sensitivity of the wearer, whereby it is possible to design an appropriate eyeglass lens according to the change in the contrast sensitivity of the wearer.

Furthermore, the first eye chart TA has the first spatial frequency f1 lower than the spatial frequency f0 of the eye chart corresponding to the visual acuity of the wearer, and the second eye chart TB has the second spatial frequency f2 lower than the first spatial frequency f1. This makes it possible to measure the contrast sensitivity with higher accuracy, using the first eye chart TA and the second eye chart TB with different spatial frequencies which are set according to the visual acuity of the wearer. The first spatial frequency f1 of the first eye chart TA and the second spatial frequency f2 of the second eye chart TB are set to numerical values higher than 8 [cycle/deg]. Thus, it is possible to measure the contrast sensitivity with higher accuracy based on a condition under which the contrast sensitivity becomes generally high.

In addition, the ordering device 60 of the eyeglass lens includes the input part 68 that inputs the design parameter Pc calculated based on the information relating to the measurement result of the contrast sensitivity of the wearer who wears the eyeglass lens, and the communication part 66 (transmitting part) that transmits the design parameter Pc input through the input part 68 to the order receiving device 70. The order receiving device 70 of the eyeglass lens includes the communication part 76 (receiving part) that receives the design parameter Pc, and the design part 73 that designs the eyeglass lens based on the design parameter Pc. Using the eyeglass lens ordering and order receiving system 50 including the ordering device 60 and the order receiving device 70, it is possible to design an appropriate eyeglass lens according to the change in the contrast sensitivity of the wearer, as described above.

In the above-described embodiment, the ordering device 60 of the eyeglass lens includes the input part 68 that inputs the design parameter Pc and the communication part 66 that transmits the design parameter Pc input through the input part 68 to the order receiving device 70, but is not limited thereto. For example, the ordering device of the eyeglass lens may include an input part that inputs the information relating to the measurement result of the contrast sensitivity of the wearer who wears the eyeglass lens, and a communication part 66 that transmits the information input through the input part to the order receiving device 70. In this case, the order receiving device of the eyeglass lens may include a communication part (receiving part) that receives the information relating to the measurement result of the contrast sensitivity of the wearer, and a design part that designs the eyeglass lens based on the information. In this case, the design part of the order receiving device may calculate the design parameter Pc based on the information relating to the measurement result of the contrast sensitivity of the wearer.

In the above-described embodiment, the first measurement of the contrast sensitivity is performed using the first eye chart TA, and the second measurement of the contrast sensitivity is performed using the second eye chart TB, but the present invention is not limited thereto. Furthermore, a third measurement of the contrast sensitivity may be performed using a third eye chart with a third spatial frequency different from the spatial frequencies of the first eye chart TA and the second eye chart TB, and measurement of the contrast sensitivity may be performed using at least two eye charts with different spatial frequencies.

In the above-described embodiment, the contrast C of the eye chart is not limited to Expression (1) exemplified above, and may be expressed by the following Expression (5).

[Formula 5]

$$C = \frac{Lmax - Lmin}{Lmax + Lmin} \quad \cdots (5)$$

Similarly to Expression (1), Lmax indicates the maximum luminance of the eye chart, and Lmin indicates the minimum luminance of the eye chart. However, it is preferable to use the same definition expression for the contrast of the eye chart in the same eyeglass lens ordering and order receiving system. In other words, it is preferable to avoid using a plurality of definition expressions for the contrast of the eye chart in the same eyeglass lens ordering and order receiving system.

EXPLANATION OF NUMERALS AND CHARACTERS

1       pair of eyeglass lenses
10R     right-eye eyeglass lens
10L     left-eye eyeglass lens
50      eyeglass lens ordering and order receiving system
60      ordering device
70      order receiving device

**Claims**

**1.** An eyeglass lens production method comprising:

performing measurement of contrast sensitivity of a wearer of an eyeglass lens using at least two eye charts with different spatial frequencies;

performing a design of the eyeglass lens based on a measurement result of the contrast sensitivity; and producing the eyeglass lens based on the design.

2. The eyeglass lens production method according to Claim 1, wherein

the at least two eye charts include a first eye chart with a first spatial frequency and a second eye chart with a second spatial frequency lower than the first spatial frequency, the measurement of the contrast sensitivity includes a first measurement that uses the first eye chart to perform the measurement of the contrast sensitivity and a second measurement that uses the second eye chart to perform the measurement of the contrast sensitivity, and the design is performed based on a measurement result of the first measurement and a measurement result of the second measurement.

3. The eyeglass lens production method according to Claim 2, wherein the first spatial frequency and the second spatial frequency are set to a numerical value higher than 8 [cycle/deg].

4. The eyeglass lens production method according to any one of Claims 1 to 3, wherein

the eyeglass lens includes a distance portion having a refractive power suitable for distance viewing, a near portion having a refractive power suitable for near viewing, and a progressive portion provided between the distance portion and the near portion and having a refractive power that changes between the refractive power of the distance portion and the refractive power of the near portion, and the design is performed such that the refractive power of the progressive portion is changed according to the measurement result of the contrast sensitivity.

5. The eyeglass lens production method according to Claim 4, wherein the design is performed based on the measurement result such that the refractive power of the progressive portion becomes high as a difference in contrast sensitivity with respect to the different spatial frequencies becomes large.

6. The eyeglass lens production method according to any one of Claims 1 to 5, wherein the different spatial frequencies are set to numerical values lower than a spatial frequency of an eye chart corresponding to visual acuity of the wearer.

7. An eyeglass lens ordering device comprising:

an input part that inputs information on a measurement result of contrast sensitivity of a wearer of an eyeglass lens which performs measurement using at least two eye charts with different spatial frequencies, or a design parameter calculated based on the information; and a transmitting part that transmits the information input through the input part or the design parameter to an eyeglass lens order receiving device.

8. An eyeglass lens order receiving device comprising:

a receiving part that receives information on a measurement result of contrast sensitivity of a wearer of an eyeglass lens which performs measurement using at least two eye charts with different spatial frequencies, or a design parameter calculated based on the information; and a design part that performs a design of an eyeglass lens based on the information or the design parameter.

9. An eyeglass lens ordering and order receiving system comprising:

the eyeglass lens ordering device according to Claim 7; and the eyeglass lens order receiving device according to Claim 8.

# FIG.1

**FIG.2**

# FIG.3

# FIG.4

ORDERING DEVICE 60

CONTROL PART 61

ORDER PROCESSING PART 62

STORAGE PART 65

COMMUNI-CATION PART 66

DISPLAY PART 67

INPUT PART 68

90

ORDER RECEIVING DEVICE 70

CONTROL PART 71

ORDER RECEIVING PROCESSING PART 72

DESIGN PART 73

STORAGE PART 75

COMMUNI-CATION PART 76

DISPLAY PART 77

INPUT PART 78

PROCESSING MACHINE CONTROL DEVICE 80

EYEGLASS LENS PROCESSING MACHINE 85

50

EP 4 643 760 A1

# FIG.5

```
   ┌──────────────┐                    ┌──────────────┐
   │    START     │                    │    START     │
   └──────┬───────┘                    └──────┬───────┘
          │                                   │
   ┌──────▼───────┐                           │
   │   MEASURE    │                           │
   │   CONTRAST   │  ST11                     │
   │ SENSITIVITY  │                           │
   └──────┬───────┘                           │
          │                                   │
   ┌──────▼───────┐                           │
   │INPUT ORDERING│  ST12                     │
   │ INFORMATION  │                           │
   └──────┬───────┘                           │
          │                                   │
   ┌──────▼───────┐  ST13     ┌───────────────▼──────┐
   │  TRANSMIT    │           │  RECEIVE ORDERING    │  ST21
   │  ORDERING    │- - - - - ▶│    INFORMATION       │
   │ INFORMATION  │           └──────────┬───────────┘
   └──────┬───────┘                      │
          │                   ┌──────────▼───────────┐
          │                   │      DESIGN          │  ST22
          │                   │   EYEGLASS LENS      │
          │                   └──────────┬───────────┘
          │                              │
          │                   ┌──────────▼───────────┐
          │                   │     PROCESS          │  ST23
          │                   │   EYEGLASS LENS      │
          │                   └──────────┬───────────┘
   ┌──────▼───────┐                ┌─────▼──────┐
   │     END      │                │    END     │
   └──────────────┘                └────────────┘
```

# FIG.6

100

106

[LENS INFORMATION]

101

| | PRODUCT NAME | S POWER | C POWER | AXIS POWER | ADDITION POWER |
|---|---|---|---|---|---|
| RIGHT | LENS A | −2.25 | −0.25 | 90 | 2.00 |
| LEFT | LENS A | −2.25 | −0.25 | 90 | 2.00 |

[PROCESSING SPECIFICATION INFORMATION]

102

| | PROCESSING SPECIFICATION | | | |
|---|---|---|---|---|
| RIGHT | OUTER DIAMETER 60 | | | |
| LEFT | OUTER DIAMETER 60 | | | |

[TINTING INFORMATION]

103

| TINTING COLOR | HOW TO TINT | CONCENTRATION |
|---|---|---|
| COLOR A | HALF | 15% |

[FITTING POINT INFORMATION]

104

| | PD | FP |
|---|---|---|
| RIGHT | 32.5 | 2 |
| LEFT | 32.5 | 2 |

[SENSITIVITY INFORMATION]

| DESING PARAMETER Pc |
|---|
| 4 |

[FRAME INFORMATION]

105

| MODEL NAME | FRAME TYPE | FRAME PD |
|---|---|---|
| | | |

EP 4 643 760 A1

# FIG.7

START

OBTAIN MINIMUM
VISIBLE EYE CHART — ST111

PERFORM FIRST
MEASUREMENT OF
CONTRAST
SENSITIVITY — ST112

PERFORM SECOND
MEASUREMENT OF
CONTRAST
SENSITIVITY — ST113

OBTAIN DESIGN
PARAMETER — ST114

END

# FIG.8

# FIG.9

| | | |
|---|---|---|
| TA1 | E K V | |
| TA2 | C O K | |
| TA3 | C N S | TA0 |
| ⋮ | | |
| TAm-1 | S C Z | |
| TAm | K O N | |

# FIG.10

| | | | |
|---|---|---|---|
| TB1 | E | K | V |

| | | | |
|---|---|---|---|
| TB2 | C | O | K |

| | | | |
|---|---|---|---|
| TB3 | C | N | S | — TB0

TB

.
.
.

| | | | |
|---|---|---|---|
| TBm-1 | S | C | Z |

| | | | |
|---|---|---|---|
| TBm | K | O | N |

# FIG.11

95

MEASURING DEVICE

96

DISPLAY DEVICE

97

DISPLAY
CONTROL PART

98

STORAGE
PART

99

INPUT
PART

# FIG.12

96

TG1 — | C N S | K O N | — TGγ

TGδ — | S C Z | C O K | — TGε

Back | OK

99a(99)

# *FIG.13*

# FIG.14

# FIG.15

EP 4 643 760 A1

# FIG.16

# FIG.17

# FIG.18

# FIG.19

START

ACQUIRE
PRESCRIPTION DATA
AND DESIGN
PARAMETER — ST221

SET TARGET
ABERRATION
OF EYEGLASS LENS — ST222

DETERMINE OVERALL
SHAPE OF LENS — ST223

ST224

DOES
OPTICAL
CHARACTERISTICS
SATISFY DESIRED
CONDITIONS
?

NO

YES

END

## INTERNATIONAL SEARCH REPORT

| International application No. |
| --- |
| **PCT/JP2023/029216** |

| | |
| --- | --- |
| **A.** | **CLASSIFICATION OF SUBJECT MATTER** |

*A61B 3/06*(2006.01)i; *G02C 7/02*(2006.01)i; *G02C 7/06*(2006.01)i; *G02C 13/00*(2006.01)i
FI:    A61B3/06; G02C7/02; G02C7/06; G02C13/00

According to International Patent Classification (IPC) or to both national classification and IPC

| | |
| --- | --- |
| **B.** | **FIELDS SEARCHED** |

Minimum documentation searched (classification system followed by classification symbols)

A61B3/06; G02C7/02; G02C7/06; G02C13/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

| | |
| --- | --- |
| **C.** | **DOCUMENTS CONSIDERED TO BE RELEVANT** |

| Category* | Citation of document, with indication, where appropriate, of the relevant passages | Relevant to claim No. |
| --- | --- | --- |
| X | JP 3210575 U (UCHIDA, Saeko) 01 June 2017 (2017-06-01) paragraphs [0001], [0015], fig. 1 | 1-3, 6-9 |
| A | | 4-5 |

☐ Further documents are listed in the continuation of Box C.    ☑ See patent family annex.

| | | | |
| --- | --- | --- | --- |
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **24 August 2023** | **03 October 2023** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915** **Japan** | |
| | Telephone No. |

Form PCT/ISA/210 (second sheet) (January 2015)

INTERNATIONAL SEARCH REPORT
Information on patent family members

International application No.

**PCT/JP2023/029216**

| Patent document cited in search report | | | Publication date (day/month/year) | Patent family member(s) | Publication date (day/month/year) |
|---|---|---|---|---|---|
| JP | 3210575 | U | 01 June 2017 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2021157001 A